# EUROPEAN PATENT APPLICATION

(11) **EP 4 810 455 A1**
(43) Date of publication of application: **23.09.2026**
(21) Application number: 24891528.2
(22) Date of filing: 15.11.2024
(51) Int. Cl.: C04B 35/115, A61C 13/083, A61K 6/802, A61K 6/824

(54) **DENTAL ALUMINA CALCINED BODY HAVING HIGH TRANSPARENCY AND HIGH STRENGTH, SINTERED BODY THEREOF, AND METHODS FOR PRODUCING SAME**

(30) Priority: 16.11.2023 JP 2023195537
(71) Applicant: Kuraray Noritake Dental Inc., Kurashiki-shi, Okayama 710-0801 (JP)
(72) Inventor: SAKAMOTO, Hiroyuki, Miyoshi-shi, Aichi 470-0293 (JP); KASHIKI, Nobusuke, Miyoshi-shi, Aichi 470-0293 (JP); MATSUURA, Atsushi, Miyoshi-shi, Aichi 470-0293 (JP)
(74) Representative: D Young & Co LLP
(86) International application number: PCT/JP2024/040725
(87) International publication number: WO 2025/105493

(57) **Abstract**

The present invention provides a dental alumina pre-sintered body capable of yielding, through sintering under atmospheric pressure, a dental alumina sintered body that exhibits reduced yellow coloration and excellent aesthetics suited for dental use while possessing superior mechanical strength along with translucency suited for dental applications. The present invention relates to a dental alumina pre-sintered body comprising alumina particles with an average primary particle diameter of 30 to 300 nm, and a sintering aid, wherein the sintering aid comprises a group 14 metallic element component, and the content of the group 14 metallic element component is more than 0 ppm and 5,000 ppm or less.

## Description

### TECHNICAL FIELD

The present invention relates to a pre-sintered body whose main component is aluminum oxide (alumina) and that becomes a highly transparent sintered body after firing, and to a highly aesthetic sintered body and methods for producing such pre-sintered bodies and sintered bodies.

### BACKGROUND ART

In recent years, sintered bodies of metal oxides have gained widespread use as dental materials. The appearance of sintered bodies used in these applications is adjusted by controlling color tone and translucency so as to conform to the appearance of healthy teeth surrounding the treated area of the patient. Generally, transparency is increased toward the incisal edge while the cervical region is adjusted to exhibit a darker color tone.

Various metal oxides are used in dental materials, including glass, zirconia, and aluminum oxide (for example, Patent Literatures 1 to 5).

### CITATION LIST

### Patent Literature

Patent Literature 1: JP2001-064075 A
Patent Literature 2: JP2010-120796 A
Patent Literature 3: JP2005-53758 A
Patent Literature 4: WO2023/127562
Patent Literature 5: US Patent Application Publication No. 2022/055948 A1

### SUMMARY OF INVENTION

### Technical Problem

For example, Patent Literature 1 uses a magnesium compound as a sintering aid, with a compound of a group 3A or group 4A metallic element added in an amount of 1 to 100 weight% relative to magnesium. However, upon examination, the present inventors have found that while the sintered body shows high transparency, residual internal pores lead to yellowness in the sintered body. This makes the invention according to Patent Literature 1 not suitable for dental use.

Patent Literature 2 describes an alumina sintered body that comprises 100 ppm to 3 wt% of a compound selected from a transition metal oxide, a group 1A alkali metal oxides, and a group 2A alkali earth metal oxide, and that exhibits a total transmittance (at a thickness of 1 mm) of 60% or more for light in the wavelength range of 300 to 800 nm. However, Patent Literature 2 gives no consideration to tetravalent tin oxide.

Moreover, Patent Literature 2 requires hot isostatic pressing following firing under atmospheric pressure, and employs transition metal oxides for coloration and anisotropic crystal growth, not as sintering aids.

In addition, the present inventors have found that the excessively high total transmittance of the sintered body poses a problem in clinical applications. Specifically, when placed in the mouth as a dental prosthesis, the sintered body appears dark in the oral cavity, causing a significant decline in the aesthetics of the dental prosthesis.

The alumina sintered body according to Patent Literature 3 requires four or more oxides selected from Si, Mn, Ti, Zr, and a group 2a element of the periodic table. However, the present inventors revealed that the alumina sintered body according to Patent Literature 3 involves residual pores, and fails to achieve both transparency and strength.

Moreover, Patent Literature 3 does not consider use in dental applications.

Patent Literature 4 requires the use of a sintering aid and a blue pigment, with the blue pigment added to counteract the yellowish hue attributed to magnesia in the sintering aid. However, it has been found by the present inventors that, despite excellent translucency, mechanical strength is insufficient in the invention according to Patent Literature 4, and the strength issue arising from residual pores within the sintered body remains unsolved.

There accordingly remain areas for improvement from the perspective of achieving both high translucency and high strength.

Patent Literature 5 describes an aluminum oxide ceramic material comprising a magnesia compound and Eu₂O₃. However, it has been found that the invention according to Patent Literature 5 involves high manufacturing costs due to the use of expensive Eu₂O₃ and a complicated manufacturing process. Another finding is that the material, when it exhibits a negative b*value, produces a markedly different hue that deviates from the range suited for dental applications, or, when the b*value exceeds 6, it imparts an excessively strong yellow tint. That is, this approach does not provide a fundamental solution to the problem of yellowish appearance due to magnesia-related residual pores within the sintered body.

As discussed above, conventional techniques employ magnesia compounds and the like as sintering aids. However, the present inventors found that the fired body exhibits a yellowish tone when sintered under atmospheric pressure.

Moreover, conventional techniques employ transition metal oxides along with group 1A alkali metal oxides and group 2A alkali earth metal oxides, and, in order to achieve high transparency, require hot isostatic pressing following sintering under atmospheric pressure.

It is accordingly an object of the present invention to provide a dental alumina pre-sintered body capable of yielding, through sintering under atmospheric pressure, a dental alumina sintered body that exhibits reduced yellow coloration and excellent aesthetics suited for dental use while possessing superior mechanical strength along with translucency suited for dental applications.

### Solution to Problem

The present inventors conducted intensive studies to find a solution to the foregoing issues, and found that yellow coloration in a sintered body sintered under atmospheric pressure can be reduced with the use of a dental alumina pre-sintered body that comprises alumina particles with an average primary particle diameter of 30 to 300 nm, and a sintering aid, and in which the sintering aid comprises a group 14 metallic element component, and the content of the group 14 metallic element component is more than 0 ppm and 5,000 ppm or less, thereby providing a sintered body that exhibits high aesthetics with translucency suited for dental applications while possessing superior mechanical strength, even when it is produced through sintering under atmospheric pressure. Building upon this finding, the present inventors conducted additional examinations, ultimately resulting in the completion of the present invention.

Specifically, the present invention includes the following.
[1] A dental alumina pre-sintered body comprising alumina particles with an average primary particle diameter of 30 to 300 nm, and a sintering aid,
   wherein the sintering aid comprises a group 14 metallic element component, and
   the content of the group 14 metallic element component is more than 0 ppm and 5,000 ppm or less.
[2] The dental alumina pre-sintered body according to [1], wherein the group 14 metallic element component is a tin component.
[3] The dental alumina pre-sintered body according to [2], wherein the tin component is an inorganic tin compound.
[4] The dental alumina pre-sintered body according to [2] or [3], wherein the tin component is at least one selected from tin(II) oxide, tin(IV) oxide, tin(VI) oxide, tin(II) chloride, tin(IV) chloride, tin(II) iodide, tin(IV) iodide, diammonium hexachlorostannate, tin(IV) sulfide, tin(II) sulfide, tin(II) sulfate, tin(IV) diphosphate, tin(II) acetate, tin(II) tartrate, potassium stannate(IV) trihydrate, and tin(II) oxalate.
[5] The dental alumina pre-sintered body according to any one of [1] to [4], wherein the group 14 metallic element component is a metal oxide.
[6] The dental alumina pre-sintered body according to any one of [1] to [5], wherein the content of the group 14 metallic element component is more than 60 ppm.
[7] The dental alumina pre-sintered body according to any one of [1] to [6], wherein the alumina particles comprise α-alumina with a purity of 99.5% or more.
[8] The dental alumina pre-sintered body according to any one of [1] to [7], which has a total transmittance of 0.3 to 5% at a thickness of 1.2 mm.
[9] A dental mill blank having a portion comprising the dental alumina pre-sintered body of any one of [1] to [8].
[10] The dental mill blank according to [9], wherein said portion is a portion with the highest translucency.
[11] A dental alumina sintered body having an average crystal grain size of 0.3 to 8.0 µm and comprising a group 14 metallic element component,
   wherein the content of the group 14 metallic element component is more than 0 ppm and 5,000 ppm or less.
[12] The dental alumina sintered body according to [11], wherein the group 14 metallic element component is a tin component.
[13] The dental alumina sintered body according to [12], wherein the tin component is at least one selected from tin(II) oxide, tin(IV) oxide, tin(VI) oxide, tin(II) chloride, tin(IV) chloride, tin(II) iodide, tin(IV) iodide, diammonium hexachlorostannate, tin(IV) sulfide, tin(II) sulfide, tin(II) sulfate, tin(IV) diphosphate, tin(II) acetate, tin(II) tartrate, potassium stannate(IV) trihydrate, and tin(II) oxalate.
[14] The dental alumina sintered body according to [12], wherein the content of the group 14 metallic element component is more than 60 ppm.
[15] The dental alumina sintered body according to [12] or [13], wherein the group 14 metallic element component is a metal oxide.
[16] The dental alumina sintered body according to any one of [12] to [15], which has a total transmittance of 40 to 65% at a thickness of 1.0 mm.
[17] The dental alumina sintered body according to any one of [12] to [16], which has a biaxial flexural strength of 580 MPa or more.
[18] A method for producing a dental alumina pre-sintered body, comprising the step of allowing a group 14 metallic element component to uniformly adhere to alumina particles,
   wherein the dental alumina pre-sintered body comprises alumina particles with an average primary particle diameter of 30 to 300 nm, and a group 14 metallic element component, and
   the content of the group 14 metallic element component is more than 0 ppm and 5,000 ppm or less.
[19] The method for producing a dental alumina pre-sintered body according to [18], which further comprises molding the alumina particles to obtain a molded body, and pre-sintering the molded body to obtain a pre-sintered body.
[20] The method for producing a dental alumina pre-sintered body according to [19], wherein the molded body is pre-sintered at a temperature of 600 to 900°C.
[21] A method for producing a dental alumina sintered body, comprising sintering the dental alumina pre-sintered body of any one of [1] to [7] at 1,500°C or less under atmospheric pressure.

### Advantageous Effects of Invention

According to the present invention, a dental alumina pre-sintered body can be provided that is capable of yielding, through sintering under atmospheric pressure, a dental alumina sintered body that exhibits reduced yellow coloration and excellent aesthetics suited for dental use while possessing superior mechanical strength along with translucency suited for dental applications.

The present invention can also provide a dental alumina pre-sintered body for producing a dental alumina sintered body that exhibits high aesthetics especially suited for the appearance of the incisal edge of incisors or canines.

In contrast to conventional techniques where yellowness intensifies with increasing thickness of the sintered body and becomes particularly pronounced at a thickness of about 5 mm, the present invention makes it possible to reduce an increase in yellow tint even with an increasing thickness of a dental alumina sintered body (for example, even in a sintered body having a thickness of about 5 mm).

Moreover, in conventional techniques, the b* value, which is associated with yellowness, cannot be readily adjusted to fall within a predetermined range and a colorant (such as a blue colorant) needs to be incorporated to reduce yellowness. However, with a dental alumina pre-sintered body of the present invention, it is possible to reduce yellowness with a b* value adjusted to fall within a desired range in the dental alumina sintered body produced, without incorporating a colorant.

With a method for producing a dental alumina sintered body of the present invention, a dental alumina sintered body can be provided that exhibits excellent mechanical strength and translucency through sintering under atmospheric pressure, without using a HIP apparatus.

### DESCRIPTION OF EMBODIMENTS

The following describes a dental alumina pre-sintered body of the present invention.

A dental alumina pre-sintered body of the present invention comprises alumina particles with an average primary particle diameter of 30 to 300 nm, and a sintering aid, wherein the sintering aid comprises a group 14 metallic element component, and the content of the group 14 metallic element component is more than 0 ppm and 5,000 ppm or less.

As used herein, "molded body" refers to a state in which neither a semi-sintered state (pre-sintered state) nor a sintered state has reached. That is, "molded body" distinguishes itself from pre-sintered body and sintered body in that it remains unfired after being molded.

As used herein, a dental alumina pre-sintered body can be a precursor (intermediate product) of a dental alumina sintered body.

As used herein, "alumina pre-sintered body" refers to a state in which particles of alumina have formed necks between particles and consolidated without reaching full sintering.

As used herein, "alumina sintered body" refers to a state in which the alumina particles (powder) have sintered.

As used herein, "atmospheric pressure" means standard atmospheric pressure (1 atm).

As used herein, "primary particles" refers to a bulk representing the smallest unit. The term "primary particles" refers to spherical particles as observed under an electron microscope (for example, a scanning electron microscope).

As used herein, "ppm" means ppm by mass.

As used herein, "sintering aid" refers to an auxiliary agent that acts to promote and stabilize sintering of alumina.

As used herein, "pre-sintering temperature" refers to the highest temperature during pre-sintering in the production of a pre-sintered body.

As used herein, "sinterable temperature" refers to the highest sintering temperature during firing in the production of a sintered body.

As used herein, "b* value" refers to chromaticity in the L*a*b* color system (JIS Z 8781-4:2013 Color Measurements - Part 4: CIE 1976 L*a*b* color space).

As used herein, "BET specific surface area" is a specific surface area measured without distinguishing primary particles and secondary particles.

As used herein, the content of a sintering aid (including the content of a group 14 metallic element component) refers to the amount of the sintering aid added in a raw material alumina powder of the present invention.

As used herein, "silicon" refers to a nonmetal, excluded from the category of metals.

In this specification, the upper limits and lower limits of numeric ranges (for example, temperature ranges, ranges of average primary particle diameters, ranges of contents of components, ranges of values calculated from components, and ranges of physical properties) can be appropriately combined.

The present invention also encompasses embodiments selected within the numerical ranges described in this specification.

A dental alumina pre-sintered body of the present invention may have a predetermined shape (for example, disc-shape or cuboidal).

A dental alumina pre-sintered body of the present invention may be a workpiece that has been processed into, for example, a crown shape. The dental alumina pre-sintered body will be referred to as "workpiece" or "cut or ground workpiece" when it has been processed. The workpiece can be obtained by, for example, processing an alumina disc (pre-sintered body) into a dental product (for example, a crown-shaped prosthesis) with a CAD/CAM (Computer-Aided Design/Computer-Aided Manufacturing) system.

A dental alumina pre-sintered body of the present invention comprises necks formed by particles of alumina (hereinafter, also referred to simply as "alumina particles"), and the extent of necking varies with the average particle diameter of the alumina particles, resulting in a change in the hardness of the dental alumina pre-sintered body.

The smaller the average primary particle diameter of the alumina particles, the more likely necking takes place during pre-sintering.

In view of achieving both high mechanical strength and high translucency after sintering and providing high aesthetics by inhibiting yellow coloration resulting from sintering, a dental alumina pre-sintered body of the present invention comprises a sintering aid, and the sintering aid comprises a group 14 metallic element component.

It has been known that heat treatment of alumina at temperatures of approximately 1,000°C or less results in a decrease in transparency due to migration and aggregation of residual pores. For this reason, sintering of alumina has conventionally been promoted through the addition of a sintering aid (preferably, a magnesium compound such as MgO).

However, with conventional techniques, it is not possible to sufficiently reduce residual pores within the sintered body under atmospheric pressure, necessitating hot isostatic pressing following sintering under atmospheric pressure.

It may be possible to reduce yellowness by reducing the content of sintering aid. However, with a reduced content, the sintering aid fails to sufficiently produce its effect, resulting in inadequate promotion of alumina sintering. In this respect, hot isostatic pressing (HIP) is also needed to sufficiently promote sintering while reducing yellowness.

In the absence of hot isostatic pressing, it has been difficult to sufficiently reduce residual pores within the sintered body by atmospheric-pressure sintering alone, making the conventional techniques inadequate in terms of achieving both high mechanical strength and high translucency in the dental alumina sintered body after sintering.

With conventional techniques employing hot isostatic pressing, it is possible to produce an alumina sintered body having a certain level of transparency. However, it was found that the presence of a trace amount of residual pores within the sintered body gives rise to a phenomenon in which light becomes increasing diffused toward the interior of the alumina sintered body, causing a yellowish appearance.

Concerning yellow coloration, a sintered body is particularly susceptible to the influence of pores remaining within its interior, and the yellow tint intensifies as the thickness increases. This has created a situation presenting difficulties in producing a dental alumina pre-sintered body that exhibits reduced yellow coloration at a thickness of approximately 5 mm.

That is, a significant challenge exists in obtaining a dental alumina pre-sintered body capable of yielding a dental alumina sintered body, approximately 5 mm thick, that exhibits reduced yellow coloration while possessing superior mechanical strength along with translucency suited for dental applications, without performing hot isostatic pressing or other such processes after sintering under atmospheric pressure.

The following speculation has been made with regard to how the dental alumina sintered body obtained through sintering of a dental alumina pre-sintered body of the present invention under atmospheric pressure exhibits reduced yellow coloration and excellent aesthetics suited for dental use while possessing superior mechanical strength along with translucency suited for dental applications.

Presumably, a heat treatment involving a temperature of approximately 1,000°C or less in the production of a dental alumina pre-sintered body of the present invention causes the group 14 metallic element component, acting as a sintering aid, to diffuse into the alumina grain boundaries, lowering the mobility of the alumina grain boundary interface and allowing the formation of a glass phase, leading to densification of the sintered body while smoothly eliminating pores present at the crystal grain boundaries, thereby significantly reducing residual pores within the sintered body.

Using a group 14 metallic element component as the sintering aid is believed to increase the diffusion distance compared to when using only MgO as the sintering aid as in conventional techniques, leading to improved transparency and reduced yellow coloration, though the crystal grain size becomes slightly larger.

Furthermore, it is believed that the marked reduction of pores remaining inside the sintered body leads to fewer fracture origins, thereby improving the mechanical strength of the dental alumina sintered body.

Examples of the group 14 metallic element component contained in a dental alumina pre-sintered body of the present invention include a germanium component, a tin component, a lead component, and a flerovium component. In view of even superior ability to eliminate pores present at the crystal grain boundaries, preferred are a germanium component and a tin component, more preferably a tin component.

The group 14 metallic element component may be used alone, or two or more thereof may be used in combination.

In view of even superior ability to eliminate pores present at the crystal grain boundaries, the tin component is preferably an inorganic tin compound.

Preferably, the inorganic tin compound is at least one selected from tin(II) oxide, tin(IV) oxide, tin(VI) oxide, tin(II) chloride, tin(IV) chloride, tin(II) iodide, tin(IV) iodide, diammonium hexachlorostannate, tin(IV) sulfide, tin(II) sulfide, tin(II) sulfate, tin(IV) diphosphate, tin(II) acetate, tin(II) tartrate, potassium stannate(IV) trihydrate, and tin(II) oxalate. In view of particularly superior ability to allow easier formation of a glass phase and smoothly eliminate pores present at the crystal grain boundaries, more preferred is at least one selected from tin(IV) oxide, tin(IV) chloride, potassium stannate(IV) trihydrate, and tin(IV) iodide.

Preferably, the germanium component is at least one selected from germanium dioxide, germanium chloride, germanium bromide, germanium iodide, germanium sulfide, germanium n-butoxide, and bis(2-carboxyethyloxogermanium) oxide, more preferably germanium dioxide.

Preferably, the group 14 metallic element component is a metal oxide. In view of even superior ability to eliminate pores present at the crystal grain boundaries, preferred are metal oxides containing divalent or tetravalent group 14 metallic elements. In view of particularly superior ability to eliminate pores present at the crystal grain boundaries, more preferred are metal oxides containing tetravalent group 14 metallic elements.

The content of the group 14 metallic element component is more than 0 ppm and 5,000 ppm or less in terms of an element equivalent (for example, Sn equivalent).

In view of even superior ability to eliminate pores present at the crystal grain boundaries, the content of the group 14 metallic element component is preferably more than 60 ppm, more preferably 100 ppm or more, even more preferably 150 ppm or more, particularly preferably 200 ppm or more.

In view of even superior ability to eliminate pores present at the crystal grain boundaries, the content of the group 14 metallic element component is preferably 4,000 ppm or less, more preferably 3,500 ppm or less, even more preferably 3,000 ppm or less, particularly preferably 2,000 ppm or less.

The sintering aid contained in a dental alumina pre-sintered body of the present invention may be used alone, or two or more thereof may be used in combination.

The sintering aid contained in a dental alumina pre-sintered body of the present invention may comprise metallic element components other than group 14 metallic element components, in addition to group 14 metallic element components (hereinafter, such metallic elements will also be referred to simply as "additional metallic element components").

A certain preferred embodiment is, for example, a dental alumina pre-sintered body in which the sintering aid is solely a group 14 metallic element component.

The additional metallic element components comprise preferably at least one element component selected from the group consisting of a group 2 element (Be, Mg, Ca, Sr, Ba, Ra), Ce, Zr, and Y, more preferably at least one element component selected from the group consisting of Mg, Ca, Sr, Ba, Ce, Zr, and Y, even more preferably at least one element component selected from the group consisting of Mg, Ce, Zr, and Y.

Most preferred as additional metallic element components are magnesium compounds.

Examples of the magnesium compounds include oxides, nitrates, acetates, hydroxides, and chlorides.

Examples of the additional metallic element components include MgCl₂, Mg(OH)₂, CeO₂, ZrO₂, and Y₂O₃.

The magnesium compound is not particularly limited, as long as it is a magnesium compound that turns into an oxide at 1,200°C or less during atmospheric sintering. Preferred examples include magnesium nitrate, magnesium chloride, magnesium hydroxide, and magnesium acetate.

The additional metallic element components may be used alone, or two or more thereof may be used in combination.

Typically, the content of the additional metallic element components in the sintering aid within a raw material alumina powder of the present invention is preferably 10 ppm or more in terms of an element equivalent (for example, Mg equivalent). In view of sufficiently promoting sintering of alumina, the content is more preferably 20 ppm or more, even more preferably 30 ppm or more.

The content of additional metallic element components is preferably 800 ppm or less. In view of allowing a further reduction of a yellow tint while promoting sintering of alumina, the content is more preferably 500 ppm or less, even more preferably 300 ppm or less.

When the content of additional metallic element components in the sintering aid falls within these ranges, it allows the sintering aid to act integrally with the group 14 metallic element component, and the dental alumina sintered body can exhibit excellent mechanical strength and translucency along with reduced yellow coloration even when sintered under atmospheric pressure.

Alumina powder has a dense oxide coating on its surface, which acts as a barrier to the atomic diffusion required for sintering. When, for example, magnesium oxide is used as an additional metallic element component in the sintering aid together with the group 14 metallic element component, such oxide coating on the alumina powder surface becomes more readily reduced owning to the high affinity of magnesium oxide for oxygen. The reduction reaction with alumina results in the formation of spinel particles (MgAl₂O₄), and the presence of these particles at the grain boundaries of alumina particles reduces the crystal grain size of the sintered body, imparting excellent translucency to the sintered body. When contained with magnesium oxide producing such effects, the group 14 metallic element component, with its longer diffusion distance than that of magnesium oxide, diffuses into alumina grain boundaries, lowering the mobility of the alumina grain boundary interface and allowing the formation of a glass phase, thereby smoothly eliminating pores present at the crystal grain boundaries. It is believed that this results in a dental alumina sintered body that, through sintering under atmospheric pressure, exhibits reduced yellow coloration and excellent aesthetics suited for dental use while possessing superior mechanical strength along with translucency suited for dental applications.

The content of the sintering aid in the alumina powder is essentially the same as the content of the sintering aid in a dental alumina pre-sintered body of the present invention and in the alumina composition described later.

In view of superior translucency, the alumina particles contained in a dental alumina pre-sintered body of the present invention have an average primary particle diameter of 30 to 300 nm.

An average primary particle diameter of less than 30 nm is not preferable because it increases yellowness in the sintered body. An average primary particle diameter exceeding 300 nm is also not preferable because an excessively large average crystal grain size due to the increased grain size in the sintered body after sintering impairs the aesthetics and mechanical strength of the sintered body as a dental material.

With an average primary particle diameter of 30 to 300 nm, larger particles are less likely to incorporate smaller particles during sintering of the pre-sintered body, reducing the occurrence of uneven sintering due to particle size differences and thereby improving translucency.

The alumina particles have an average primary particle diameter of preferably 40 to 250 nm, more preferably 60 to 200 nm, even more preferably 80 to 150 nm.

The method for measuring the average primary particle diameter in the pre-sintered body is as described in the EXAMPLES below. The number of alumina particles measured for determining the average primary particle diameter in the dental alumina pre-sintered body may be 100 or more, 200 or more, or 600 or more (for example, 600 or 800).

Because it is possible to reduce yellow coloration resulting from sintering, a dental alumina pre-sintered body of the present invention is not required to contain a blue colorant (a pigment or a composite pigment) that imparts a blue color (hereinafter, referred to as "blue colorant") to mitigate a yellow tint. Examples of such blue colorants include a Co component (a cobalt-containing compound with a composite pigment) Because there is no need for a blue colorant, a dental alumina pre-sintered body of the present invention does not require a pigment, such as a Cr component, used with a blue colorant.

A certain embodiment is, for example, a dental alumina pre-sintered body containing 0 ppm of a blue colorant (such as a cobalt component).

Because the main component of a dental alumina pre-sintered body of the present invention is alumina, the dental alumina sintered body can exhibit high aesthetics as a dental material while possessing excellent chemical stability. Here, "main component" refers to the component with the highest content. The content of the main component (alumina) may be, for example, 50 mass% or more, 70 mass% or more, 80 mass% or more, or 90 mass% or more.

The alumina particles contained in a dental alumina pre-sintered body of the present invention are more preferably α-alumina with a purity of 99.5% or more because of its low impurity content, and the ability to reduce the impurity-related formation of a glass phase at crystal grain boundaries, preventing the coarsening of crystal grains (grains), and inhibiting a decrease of aesthetics in the sintered body as a dental material.

Using α-phase aluminum oxide (α-alumina) as the starting raw material is preferable because, with its high corrosion resistance and its stability at elevated temperatures, it enables uniform control of the pre-sintered body, and the maintenance of high translucency and total transmittance after firing. Using this material is also preferred in view of enabling densification by preventing coarsening of grain size in the crystalline structure within the sintered body.

Considering these factors, it is particularly preferable that the alumina particles contained in a dental alumina pre-sintered body of the present invention comprise α-alumina with a purity of 99.5% or more.

The alumina raw material can be acquired using methods, for example, such as the alkoxide method, modified Bayer method, ammonium alum pyrolysis method, and ammonium dawsonite pyrolysis method, preferably the alkoxide method. The alkoxide method allows for enhancement of purity in a powder of alumina raw material, making it easier to achieve a uniform particle size distribution. As a specific example, aluminum hydroxide obtained through hydrolysis of purified aluminum alkoxide is fired in air at 1,100°C or higher temperatures.

Examples of the alumina raw material include α-alumina with a purity 99.99% or more, for example, such as the AKP grade (α-alumina) and the NXA grade (e.g., NXA-100, NXA-150) manufactured by Sumitomo Chemical Co., Ltd. (These are all ultrafine α-alumina.)

The content of the sintering aid in a dental alumina pre-sintered body of the present invention and in a sintered body thereof can be measured by, for example, inductively coupled plasma (ICP) emission spectral analysis, X-ray fluorescence analysis (XRF), scanning or transmission electron microscopy (SEM or TEM), energy dispersive X-ray analysis or wavelength dispersive X-ray analysis (EDX or WDX), or field emission electron probe microanalysis (FE-EPMA).

A certain embodiment is, for example, a dental alumina pre-sintered body that shows a total transmittance of 0.3% or more and 5% or less at a thickness of 1.2 mm.

The total transmittance shares the same measurement method and preferred ranges as the total transmittance of the alumina sintered body described later.

The total transmittance of the dental alumina pre-sintered body of 1.2 mm thickness is preferably 0.3% or more, more preferably 0.4% or more, even more preferably 0.5% or more.

The total transmittance of the dental alumina pre-sintered body of 1.2 mm thickness is preferably 5% or less, more preferably 4% or less, even more preferably 3% or less.

Another certain embodiment is, for example, a dental alumina pre-sintered body that shows a b* value of 0 or more and less than 6.0 in a sintered body of 1.2 mm thickness sintered under atmospheric pressure without a hot isostatic pressing process (HIP). In the present invention, the b* value is preferably 0 or more because a b* value below 0 (a negative value) results in a markedly different hue.

The b* value is preferably 0 or more, more preferably 0.1 or more, even more preferably 0.2 or more, particularly preferably 0.3 or more.

The b* value is preferably less than 6.0, more preferably less than 4.0, even more preferably less than 3.5, particularly preferably less than 3.0.

The method of measurement of b* value is as described in the EXAMPLES below.

The following describes a composition (hereinafter, also referred to as "alumina composition") for producing a dental alumina pre-sintered body of the present invention.

The alumina composition comprises alumina and a sintering aid. The sintering aid comprises a group 14 metallic element component, and the content of the group 14 metallic element component is more than 0 ppm and 5,000 ppm or less. The alumina and sintering aid may be the same alumina and sintering aid exemplified for the alumina pre-sintered body.

The alumina composition becomes a precursor of the dental alumina pre-sintered body of the present invention described above.

The alumina composition and molded body refer to a state before firing, and involve no necking between alumina particles.

The content of the alumina and sintering aid in an alumina composition of the present invention is calculated from their content in a predetermined alumina pre-sintered body, and is essentially the same for the alumina composition and alumina pre-sintered body.

An alumina composition of the present invention is not limited to particular forms, and may have various forms, including a powder, a fluid with a powder added to solvent, and a molded body of a powder formed into a predetermined shape. When an alumina composition of the present invention has a powder form, it may be a cluster of granules. The granules are formed by the aggregation of primary particles.

Preferably, the particles constituting the granules formed of the alumina composition are predominantly primary particles. Aggregates of primary particles are referred to as secondary particles. Preferably, the number of primary particles is greater than the number of secondary particles in visual confirmation through, for example, an electron microscope. Secondary particles are typically irregular in shape, and an increase in their quantity leads to sparse density during the press molding process in producing a molded body, resulting in a decrease of aesthetics due to the sparse density after sintering.

The particle diameter of primary particles within the particles constituting the granules formed of the alumina composition influences the extent of necking during pre-sintering, and affects the hardness of the dental alumina pre-sintered body.

Particles with an average primary particle diameter of less than 30 nm are not preferable because it results in strong necking due to changes in the BET specific surface area of primary particles contained in the dental alumina pre-sintered body, and a hardness increase during machining. Particles with an average primary particle diameter of more than 300 nm are not preferable because the incorporation of smaller particles in the particle size distribution becomes more likely, causing localized necking due to particle size differences, thereby increasing the occurrence of coarse density.

The average primary particle diameter is preferably 30 to 300 nm, more preferably 40 to 250 nm, even more preferably 60 to 200 nm.

The primary particles within the particles constituting the granules formed of the alumina composition may be a combination of two types of alumina particles having different average primary particle diameters. For example, when the NXA is used, an example is a combination of NXA-100 (ultrafine α-alumina manufactured by Sumitomo Chemical Co., Ltd.) and NXA-150 (ultrafine α-alumina manufactured by Sumitomo Chemical Co., Ltd.).

The particles constituting the granules formed of the alumina composition has a BET specific surface area of preferably 5 m²/g or more, more preferably 7.5 m²/g or more, even more preferably 8 m²/g or more, as measured in compliance with JIS Z 8830:2013.

With a BET specific surface area of 5 m²/g or more, it is easier to lower the sinterable temperature. This facilitates easier sintering, or makes it easier to inhibit the reduction in translucency due to the opacification occurring in the sintered body after sintering.

The BET specific surface area is preferably 25 m²/g or less, more preferably 22 m²/g or less, even more preferably 18 m²/g or less.

With a BET specific surface area of 25 m²/g or less, the average primary particle diameter is not excessively small, reducing the occurrence of coarse density in the dental alumina pre-sintered body, providing even superior aesthetics after sintering.

With a BET specific surface area of 25 m²/g or less, the pre-sintered body can be prevented from becoming overly hard. This facilitates a reduction in the amount of wear in tools (hereinafter, also referred to as "amount of tool wear") and/or the frequency of chipping (hereinafter, also referred to as "chipping rate"), or reduces the occurrence of coarse density by ensuring sufficient necking, facilitating a reduction in chipping rate.

Concerning the BET specific surface area of the dental alumina pre-sintered body and the BET specific surface area of the composition described above, the extent of necking is preferably such that the numerical difference obtained by subtracting the BET specific surface area of the pre-sintered body from the BET specific surface area of the composition is within 10 m²/g. This makes it possible to desirably maintain cutting and grinding properties.

An alumina composition of the present invention may employ a granular form in preferably 50% or more, more preferably 70% or more, even more preferably 80% or more, particularly preferably 90% or more of the alumina in the alumina composition.

When an alumina composition of the present invention does not employ a granular form, the alumina particles constituting the powder may have the average particle diameter and the BET specific surface area noted above.

The average particle diameter of granules in an alumina composition of the present invention (secondary particle diameter; hereinafter, also referred to as "average granule diameter") is preferably 10 µm or more, more preferably 12 µm or more, even more preferably 14 µm or more.

When the average granule diameter is less than 10 µm, there is a risk of trapping air in the process of placing the granules in a mold, leading to inadequate degassing during molding. This may result in the inability to fabricate a uniform and dense molded body. Additionally, there is a risk of granules being ejected through gaps during molding, potentially resulting in a molded body that does not meet the predetermined quantitative requirements. The average granule diameter is preferably 200 µm or less, more preferably 190 µm or less, even more preferably 180 µm or less, particularly preferably 150 µm or less, most preferably 100 µm or less.

When the average granule diameter exceeds 200 µm, there is an increased likelihood of cavity formation within the granules. Additionally, voids are more likely to occur when placing the granules in a mold. These phenomena pose a risk of inadequate degassing during molding, potentially resulting in the inability to fabricate a dense molded body. There is also a risk of increased shrinkage during molding, potentially leading to the inability to fabricate a molded body of the desired size.

It is preferable that 50% or more of the alumina in the alumina composition constitute granules. Preferably, the average granule diameter is measured using a method that does not disrupt the granules. The average granule diameter can be measured using a method, for example, such as dry sieving or wet sieving.

Measurement by dry sieving can be conducted following the test sieving described in JIS Z 8815:1994. Both manual sieving and mechanical sieving are applicable; however, mechanical sieving is preferred.

For sieving, the test sieve described in JIS Z 8801-1:2019 can be used.

Ro-Tap sieve shakers or sonic sieving analyzers may be used as measurement instruments for sieving, for example. Examples of the Ro-Tap sieve shakers include RPS-105M manufactured by Seishin Enterprise Co., Ltd. Examples of the sonic sieving analyzers include Robot Sifter RPS-01 and Robot Sifter RPS-02 manufactured by Seishin Enterprise Co., Ltd.

It is preferable that granules in an alumina composition of the present invention have high sphericity. By increasing the sphericity of granules, it is possible to promote mixing at the interface between layers when stacking alumina powders of different compositions. With increased sphericity, it is also possible to increase the packing density of when an alumina powder is filled into a mold to prepare a molded body, even when the average particle diameter is the same. The strength and translucency of the sintered body can increase by increasing the packing density, which is a density of a molded body formed into a specific shape under pressure after alumina granules are filled into a specific molding tool (such as a mold). It is also possible to more easily fill granules into angular portions when the mold has angular portions.

The sphericity of granules in an alumina composition of the present invention can be represented by parameters, for example, such as light bulk density, and heavy bulk density.

In view of increasing granule flow (ease of loading) to reduce coarseness in the density of the molded body obtained, an alumina composition of the present invention has a light bulk density of preferably 0.6 g/cm³ or more, more preferably 0.7 g/cm³ or more, even more preferably 0.8 g/cm³ or more, particularly preferably 0.9 g/cm³ or more.

The light bulk density can be measured in compliance with JIS R 9301-2-3:1999.

In view of increasing granule flow (ease of loading) to reduce coarseness in the density of the molded body obtained, an alumina composition of the present invention has a heavy bulk density of preferably 0.8 g/cm³ or more, more preferably 0.9 g/cm³ or more, even more preferably 1.0 g/cm³ or more.

The heavy bulk density can be measured in compliance with JIS R 9301-2-3:1999.

An alumina composition of the present invention preferably comprises a binder.

The binder may be, for example, an organic binder.

Examples of the organic binder include common binders such as acrylic binders, acrylate binders, paraffinic binders, fatty acid binders, and polyvinyl alcohol binders. Preferred among these organic binders is one having a carboxyl group in the molecular chain, or a derivative of carboxylic acids, more preferably an acrylic binder, even more preferably a polyacrylate having water solubility. The polyacrylate may be a product of copolymerization of acrylic acid or methacrylic acid with maleic acid, and may contain sulfonic acid. Examples of the cations of the salt include sodium and ammonium.

Depending on the content of the binder in an alumina composition of the present invention, it is possible to adjust the distance between primary particles in the alumina composition, and the cumulative distribution of pores, making it easier to increase or decrease the Vickers hardness or the mechanical strength of the dental alumina pre-sintered body.

The binder content is preferably 1.2 to 2.8 mass%, more preferably 1.5 to 2.5 mass%, even more preferably 1.8 to 2.2 mass% in the whole alumina composition.

When the binder content is 1.2 mass% or more in the whole alumina composition, the dental alumina pre-sintered body does not become overly strong in mechanical strength, eliminating the risk of excessive hardness when removing the cut workpiece.

When the binder content is 2.8 mass% or less, the mechanical strength of the dental alumina pre-sintered body does not overly decrease, reducing the possibility of the workpiece detaching from the pre-sintered body during cutting, in addition to facilitating a reduction in chipping rate.

An alumina composition of the present invention may optionally comprise additives other than sintering aids (additives excluding CeO₂, ZrO₂, and Y₂O₃), such as colorants (including pigments, composite pigments, and fluorescent agents), titanium oxide (TiO₂), silica (SiO₂), dispersants, and antifoaming agents. These components may be used alone, or two or more thereof may be used in combination.

Examples of the pigments include oxides of at least one element selected from the group consisting of Ti, V, Cr, Mn, Fe, Ni, Zn, Y, Zr, Sb, Bi, Ce, Sm, Nd, Eu, Gd, and Er.

A certain embodiment is, for example, an alumina composition that comprises neither a Nd-containing oxide (for example, Nd₂O₃) nor a Eu-containing oxide (for example, Eu₂O₃).

Examples of the composite pigments include (Zr,V)O₂, and Fe(Fe,Cr)₂O₄.

Examples of the fluorescent agents include Y₂SiO₅:Ce, Y₂SiO₅:Tb, (Y,Gd,Eu)BO₃, Y₂O₃:Eu, YAG:Ce, ZnGa₂O₄:Zn, and BaMgAl₁₀O₁₇:Eu.

The additives may be added during mixing or pulverization, or may be added after pulverization.

A certain embodiment is, for example, a dental mill blank having a portion comprising the dental alumina pre-sintered body.

By having a portion comprising the alumina pre-sintered body, the dental mill blank can exhibit the high level of aesthetics required for the incisal edge of incisors or canines, without the need for complementation by other materials (for example, porcelain), due to the high translucency and reduced yellow coloration exhibited by the alumina pre-sintered body following sintering. The location where such a portion is situated in the dental mill blank can be adjusted during processing, and is not specified.

Another certain embodiment is, for example, a dental mill blank in which said portion is a portion with the highest translucency.

With said portion representing a portion with the highest translucency, the dental mill blank can exhibit particularly high aesthetics when this portion is processed into the incisal region of incisors or canines. The location where such a portion is situated in the dental mill blank can be adjusted during processing, and is not specified.

A certain embodiment is, for example, a method for producing a dental alumina pre-sintered body comprising the step of allowing a group 14 metallic element component to uniformly adhere to alumina particles,
wherein the dental alumina pre-sintered body comprises alumina particles with an average primary particle diameter of 30 to 300 nm, and a sintering aid,
the sintering aid comprises a group 14 metallic element component, and
the content of the group 14 metallic element component is more than 0 ppm and 5,000 ppm or less.

The step of allowing a group 14 metallic element component (for example, a tin component) to uniformly adhere to alumina particles (hereinafter, also referred to as "adhering step") may involve, for example, dispersing alumina particles in a dispersion medium with the group 14 metallic element component (for example, a tin component) dissolved in the dispersion medium. With alumina particles dispersed in such a dispersion medium, the group 14 metallic element component (for example, a tin component) can uniformly adhere to alumina particles.

The dispersion medium is not particularly limited, and may be, for example, an organic solvent (e.g., alcohol-based solvents such as methanol and ethanol). The dispersion medium may be used alone, or two or more thereof may be used in combination.

The dispersion method is not particularly limited, and may employ known methods (for example, sonication).

By allowing the group 14 metallic element component to uniformly adhere to alumina particles, the dental alumina pre-sintered body can exhibit particularly high aesthetics when processed into the incisal region of incisors or canines.

The method for producing the dental alumina pre-sintered body may comprise the step of obtaining a molded body by pressure molding of the alumina composition obtained in the adhering step.

Another embodiment is, for example, a method for producing a dental alumina pre-sintered body comprising molding the alumina particles to obtain a molded body, and pre-sintering the molded body to obtain a pre-sintered body, in addition to the step of allowing a group 14 metallic element component to uniformly adhere to alumina particles.

Another example of an alumina pre-sintered body production method comprises the steps of:
producing an alumina composition comprising alumina particles, and a sintering aid containing a group 14 metallic element component; and
firing (pre-sintering) the alumina composition (for example, a molded body) to obtain an alumina pre-sintered body in which the alumina particles within the pre-sintered body have an average primary particle diameter of 30 to 300 nm, and the content of the sintering aid is more than 0 ppm and 5,000 ppm or less.

The process of producing an alumina composition of the present invention is described first.

First, alumina and a sintering aid containing a group 14 metallic element component are mixed in predetermined proportions to prepare a mixture (mixing step). For example, when the sintering aid is tin(IV) oxide, alumina and tin(IV) oxide may be mixed at a mixing ratio that provides the foregoing contents. The mixing process may be dry mixing or wet mixing.

Subsequently, the alumina composition may be pulverized (preferably, deagglomerated) until the foregoing average particle diameter is achieved (pulverization step).

The adhering step may be performed before and after the mixing step.

The mixing and pulverization may be performed in a single step. Pulverization can be performed by using, for example, a ball mill or bead mill after dispersing the composition and a binder in a solvent such as water or alcohol (dispersing step). The composition is pulverized (preferably, deagglomerated) to achieve an average primary particle diameter of, for example, 0.05 µm to 0.3 µm in the composition. For particle size adjustment, the composition may optionally be subjected to other processes (classification, elutriation).

The average primary particle diameter can be measured by laser diffraction/scattering particle size distribution analysis. For example, the average primary particle diameter can be measured by volume with ultrasonic waves being applied after a slurry diluted with water is subjected to 30 minutes of ultrasonication, using a laser diffraction/scattering particle size distribution analyzer (manufactured by Horiba Ltd. under the trade name Partica LA-950).

After the mixing and/or pulverization step, the mixture may be dried by spray drying with a spray dryer or the like to achieve the aforementioned granular form in the alumina composition (drying step).

In the pulverization step, the average particle diameter of the alumina composition is preferably 0.3 µm or less, more preferably 0.25 µm or less, even more preferably 0.2 µm or less, particularly preferably 0.15 µm or less. When the average particle diameter of the alumina composition is less than 0.15 µm, the sintered body can exhibit enhanced aesthetics by retaining the small crystal grain size. These average particle diameters are also preferable in terms of improved machinability.

Alumina and the sintering aid may be separately prepared. For example, it is possible to independently provide separate preparation steps (for example, production steps) for alumina and the sintering aid containing a group 14 metallic element component, instead of simultaneously precipitating alumina and the sintering aid containing a group 14 metallic element component (in the same step). In this way, the aforementioned α-alumina can be obtained with high purity and a small primary particle diameter.

Typically, in the production of alumina compositions, the sintering aid containing a group 14 metallic element component may be reacted with alumina through heat treatment, and the resulting product may be used for the pulverization and drying steps.

This enables the production of granules formed of the alumina composition, which serves as the raw material of alumina pre-sintered bodies.

The alumina composition, in granule or powder form, can be press molded into a molded body by applying an external force.

The molding method is not limited to specific methods, and a suitable method may be appropriately selected according to intended use. For example, molding may be achieved by press molding, injection molding, stereolithography, slip casting, gel casting, filtration casting, or casting. Molding may be performed in multiple stages. For example, the alumina composition may be subjected to a CIP process after press molding, or press molding and CIP molding may be repeated.

Examples of the press molding method include a uniaxial pressing process (hereinafter, also referred to as "uniaxial pressing"), a biaxial pressing process, and a CIP (Cold Isostatic Pressing) process. These may be performed in combination, as needed.

A molded body of the present invention may have a disc shape, a cuboidal shape, or a shape of a dental product (for example, a crown shape).

A certain embodiment is, for example, an alumina pre-sintered body production method in which the press molding is uniaxial pressing, and the surface pressure of uniaxial pressing is 5 MPa or more. The press molding may yield, for example, a columnar molded body packed by uniaxially pressing alumina granules filled into a mold. The molded body can increase its density with higher surface pressure during press molding. Conversely, a hard dental alumina pre-sintered body results when the density of the molded body is excessively high. For this reason, the surface pressure in press molding is preferably 5 to 600 MPa, more preferably 10 to 400 MPa, even more preferably 15 to 200 MPa. When the surface pressure of pressing is 5 MPa or more, the molded body exhibits superior shape retention. With a surface pressure of 600 MPa or less, it is possible to prevent excessive density increase in the molded body, making it easier to avoid hardening.

A molded body of the present invention encompasses molded bodies compacted by a high-temperature pressing process such as a CIP (Cold Isostatic Pressing) process. In view of the above, the hydraulic pressure is preferably 50 to 1,000 MPa, more preferably 100 to 600 MPa, even more preferably 150 to 300 MPa.

A dental alumina pre-sintered body according to the present invention is obtained by pre-sintering the molded body.

The content of the alumina and the sintering aid containing a group 14 metallic element component in a dental alumina pre-sintered body of the present invention is essentially the same as that in the alumina composition to be fabricated into an alumina pre-sintered body.

The firing temperature (hereinafter, also referred to as "pre-sintering temperature") in the step of firing under atmospheric pressure in the pre-sintered body production method (pre-sintering step) affects the Vickers hardness, or the mechanical strength of the pre-sintered body.

The dental alumina pre-sintered body undergoes changes in cumulative pore distribution and hardness with the pre-sintering temperature, leading to changes in the amount of tool wear and/or chipping rate.

In view of this, the pre-sintering temperature in a dental alumina pre-sintered body production method of the present invention is preferably 600°C or more and 900°C or less, more preferably 650°C or more and 850°C or less, even more preferably 700°C or more and 800°C or less.

When the pre-sintering temperature is 600°C or more, the pillar (support or sprue) that connects a section of the pre-sintered body to the workpiece while it is being cut or ground by machining the pre-sintered body can remain unbroken, preventing the workpiece from being detached during the cutting or grinding process. Additionally, the Vickers hardness can be adjusted within the desired range to reduce an increase in chipping rate.

When the pre-sintering temperature is 900°C or less, there is no excessive neck formation, preventing the workpiece from becoming too hard. This facilitates a quicker separation of the workpiece from the frame securing the workpiece. Additionally, an increase in chipping rate can be reduced as tool wear remains low, allowing for an easy separation of the workpiece from the pillar.

It is preferable to maintain the highest pre-sintering temperature for a certain time period because it confines the hardness of the dental alumina pre-sintered body within the preferred range, and may lead to a reduction in chipping rate.

The pre-sintering conditions depend on the average primary particle diameter of the dental alumina pre-sintered body, and the density of the pre-sintered body. The hold time at the highest pre-sintering temperature is preferably 30 minutes to 6 hours. Preferably, the rate of temperature increase to the highest pre-sintering temperature, and the rate of temperature decrease from the highest pre-sintering temperature are 300°C/min or less.

A dental alumina pre-sintered body of the present invention can be machined to fabricate a workpiece.

The machining method is not limited to specific methods, and a suitable method may be appropriately selected according to intended use. For example, an alumina disc (pre-sintered body) can be milled or ground into a shape of a dental product (for example, a crown-shaped prosthesis) with a CAD/CAM system to fabricate a workpiece.

The surface smoothness of the workpiece can be improved using tools such as polishers (for example, Pearl Surface^{®} manufactured by Kuraray Noritake Dental Inc.).

In a dental alumina pre-sintered body of the present invention, the relative density can be controlled by the method of production described later.

A relative density of less than 43% means that the proportion of pores inside the pre-sintered body is high, leading to increased chipping due to sparsity in the relative density inside the pre-sintered body. This sparsity results in uneven shrinkage rates during sintering, causing some deformation in the sintered body, increasing the need for reworking. In view of the aesthetics of the sintered body, it is not preferable to have sparsity in relative density. This is because, though the presence of sparsity may improve processability such as cutting and grinding properties, a high proportion of pores within the pre-sintered body means that the distance between particles is large, resulting in the inability to completely release voids out of the sintered body during the sintering process, leading to a decrease in the aesthetics of the sintered body.

A relative density of 43% to 63% is preferable because it provides a good overall balance between particles and pores, enabling a reduction in the amount of tool wear and/or chipping rate, and the maintenance of high aesthetics in the sintered body. The relative density is preferably 43% to 63%, more preferably 50% to 55%.

The relative density of the dental alumina pre-sintered body can be calculated from the porosity of the pre-sintered body. Specifically, a mercury porosimeter can be used for the measurement and calculation of relative density. The mercury porosimeter is preferably one capable of applying a mercury pressure of 15 to 30,000 psia, preferably 0.5 to 60,000 psia.

In view of reducing measurement errors, the preferred pressure resolution is 0.1 psia or higher.

Examples of the mercury porosimeter include AutoPore^{®} IV 9500 manufactured by Micromeritics (USA).

The density of the dental alumina pre-sintered body is defined as the density of a dental alumina pre-sintered body obtained after granules resulting from drying of raw materials are filled into a specific molding tool (such as a mold) and molded into a specific shape under pressure, and the resulting molded body is subsequently heated to remove binders at a temperature that allows for removal of binders, and heated again at a temperature that allows for a moderate level of necking.

The temperature for the removal of binders is not particularly limited as long as it allows for removal of binders, and may be 150 to 500°C. The temperature at which moderate neck formation occurs is preferably 700 to 1,200°C.

A dental alumina pre-sintered body of the present invention exhibits a change in BET specific surface area with the average primary particle diameter, the state of necking, and density.

The BET specific surface area can be measured in compliance with JIS Z 8830:2013.

For the measurement of BET specific surface area, commercially available products can be used, such as a full automatic specific surface area analyzer (manufactured by Mountech Co., Ltd. under the trade name Macsorb^{®} HM Model-1200, BET flow method (single point/multi point)).

In view of decreasing the amount of tool wear and chipping rate, a dental alumina pre-sintered body of the present invention has a BET specific surface area of preferably 5 m²/g or more, more preferably 7.5 m²/g or more, even more preferably 8 m²/g or more.

With a BET specific surface area of 5 m²/g or more, the average primary particle diameter is not excessively large, allowing for the reduction of an increase in chipping rate, or the prevention of excessive necking, thereby reducing an increase in the amount of tool wear.

The BET specific surface area is preferably 25 m²/g or less, more preferably 22 m²/g or less, even more preferably 18 m²/g or less.

With a BET specific surface area of 25 m²/g or less, the average primary particle diameter is not excessively small, preventing the dental alumina pre-sintered body from becoming overly hard. This facilitates a reduction in the amount of tool wear and/or chipping rate, or allows for a reduction of density coarseness by ensuring sufficient necking, making it easier to reduce the chipping rate.

Concerning the processability of a dental alumina pre-sintered body of the present invention, the processability is also influenced by the mechanical strength of the dental alumina pre-sintered body. The mechanical strength of a dental alumina pre-sintered body according to the present invention can be evaluated by, for example, measuring the flexural strength of the pre-sintered body.

The three-point flexural strength of a dental alumina pre-sintered body according to the present invention can be measured in compliance with JIS R 1601:2008.

In order to ensure strength that enables machining, the dental alumina pre-sintered body has a three-point flexural strength of preferably 10 MPa or more, more preferably 18 MPa or more, even more preferably 20 MPa or more.

When the dental alumina pre-sintered body has a three-point flexural strength of less than 10 MPa, the pillar (support or sprue) becomes more likely to break during the cutting or grinding process, increasing the possibility of the workpiece separating from the pre-sintered body before it becomes a cut or ground workpiece.

For easier machining, the dental alumina pre-sintered body has a three-point flexural strength of preferably 50 MPa or less, more preferably 45 MPa or less, even more preferably 40 MPa or less, particularly preferably 35 MPa or less.

In view of reducing the amount of tool wear or chipping rate, and enabling easy separation of the cut or ground workpiece from the fixture frame for quick removal while reducing tool wear, a dental alumina pre-sintered body of the present invention has a Vickers hardness of 350 HV 5/30 or less, preferably 300 HV 5/30 or less, more preferably 100 HV 5/30 or less. A Vickers hardness of less than 30HV 5/30 leads to an increase in chipping rate, whereas the amount of tool wear increases with a Vickers hardness of more than 135 HV 5/30.

Here, "HV 5/30" means a Vickers hardness under the application of a load (test force) of 5 kgf for a duration of 30 seconds.

A dental alumina pre-sintered body of the present invention can have a reduced probability of chipping with the Vickers hardness falling in the foregoing predetermined ranges.

The Vickers hardness in the present invention is measured using a method in compliance with JIS Z 2244:2020, as will be described later in detail in the EXAMPLES below.

The Vickers hardness in a dental alumina pre-sintered body of the present invention becomes more easily achievable with factors such as the average primary particle diameter of particles contained in the pre-sintered body, the relative density of the pre-sintered body, and the mechanical strength of the pre-sintered body due to the state of particle necking.

The production method of a dental alumina pre-sintered body and the production method of an alumina composition are important for attaining these factors. Specifically, important parameters include the method of production of an alumina composition that essentially does not involve the firing step, the binder content in producing the alumina composition, the surface pressure during the fabrication of the molded body, and the pre-sintering temperature in the production of the pre-sintered body, as described above.

The processing machine used for machining a dental alumina pre-sintered body of the present invention is not particularly limited. For example, milling or grinding machines such as desktop processing machines and large machining centers (general-purpose processing machine) may be used, depending on the workpiece to be processed. Examples of such milling machines include tabletop processing machines, for example, such as DWX-50, DWX-4, DWX-4W, DWX-52D, and DWX-52DCi (manufactured by Roland DG Corporation).

The tools employed in the processing machines for machining a dental alumina pre-sintered body of the present invention are not particularly limited. Milling burs and grinding burs recommended by processing machine suppliers can be suitably used. A KATANA^{®} drill represents an example of milling burs used for milling machines.

The lifetime of tools used in machining equipment is contingent on usage conditions. For example, when cutting or grinding a dental alumina pre-sintered body, wearing of the drill blade (tool wear) may lead to problems such as the processed surface of the dental alumina pre-sintered body fracturing into small pieces (chipping) or large pieces. This results in productivity-related issues such as a time-consuming rework of the machining process. Chipping is particularly prominent in traditional pre-sintered bodies.

To assess tool life, torque on the machine side can be monitored, and a specific torque value may be established as the upper threshold limit, serving as an indicator to determine tool life (an indicator to decide when the tool should be replaced). Alternatively, the processing time may serve as the upper threshold limit. Verification of tool life may involve, for example, measuring the wear width of the blade on the milling bur used in the cutting or grinding machine. In the case of a KATANA^{®} drill for example, the tool life (replacement time) can be identified when the wear width reaches 0.21 mm or more. The wear width of the blade is preferably within 0.2 mm, more preferably within 0.15 mm, even more preferably within 0.1 mm.

A dental alumina sintered body of the present invention can be fabricated by sintering a dental alumina pre-sintered body of the present invention, and a cut or ground workpiece thereof, at a temperature that sinters the alumina particles (sintering step).

The sinterable temperature (highest sintering temperature) is preferably 1,300°C or more, and may vary with the average primary particle diameter. When the average primary particle diameter is approximately 100 nm, the sinterable temperature is, for example, preferably 1,300°C or more, more preferably 1,350°C or more, even more preferably 1,375°C or more.

The sinterable temperature is, for example, preferably 1,500°C or less, more preferably 1,450°C or less. The rate of temperature increase and the rate of temperature decrease are preferably 300°C/min or less.

A certain preferred embodiment is, for example, a method for producing a dental alumina sintered body in which a dental alumina pre-sintered body of the present invention is sintered at 1,500°C or less under atmospheric pressure.

In the sintering step, the hold time at the sinterable temperature is preferably less than 120 minutes, more preferably 90 minutes or less, even more preferably 75 minutes or less, yet more preferably 60 minutes or less, particularly preferably 45 minutes or less, most preferably 30 minutes or less. The hold time is preferably 1 minute or more, more preferably 3 minutes or more, even more preferably 5 minutes or more.

An alumina composition and an alumina pre-sintered body of the present invention enable a shorter sintering process to be employed for the fabrication of a sintered body, without causing a decrease in the translucency and strength of the alumina sintered body fabricated. Particularly, it is possible to shorten the hold time at the highest sintering temperature for the fabrication of a sintered body (short sintering). This enhances production efficiency, and when a dental alumina pre-sintered body of the present invention is applied to dental products, the patient can experience less time-related stress because the time required from deciding the product dimensions and cutting or grinding the dental product to making the dental product ready for treatment can be reduced. It is also possible to reduce the energy cost.

In the sintering step, the hold time at the sinterable temperature (for example, highest sintering temperature) may be, for example, 25 minutes or less, 20 minutes or less, or 15 minutes or less.

Preferably, the rate of temperature increase to the highest sintering temperature and the rate of temperature decrease from the highest sintering temperature in the sintering process are set so as to reduce the time required for the sintering process. For example, the rate of temperature increase may be set so that the highest sintering temperature is reached in the shortest possible time, depending on the capabilities of the furnace.

The rate of temperature increase to the highest sintering temperature may be, for example, 10°C/min or more, 50°C/min or more, 100°C/min or more, 120°C/min or more, 150°C/min or more, or 200°C/min or more. Preferably, the rate of temperature decrease from the highest sintering temperature is set to a rate that does not cause deformation due to shrinkage rate differences, or defects such as cracks, in the sintered body.

As an example of the cooling process in the sintering step, the sintered body may be allowed to cool at room temperature after the heating is finished.

The following describes a dental alumina sintered body obtained by sintering a dental alumina pre-sintered body of the present invention or a workpiece thereof.

The dental alumina sintered body has a relative density of preferably 99.5% or more.

The relative density in the dental alumina sintered body can be calculated as a ratio of the actual density, measured by the Archimedes method, with respect to the theoretical density.

By "relative density", it means a value obtained by theoretically dividing density d1 by density d2, where d1 is the density of a sintered body after high-temperature sintering of a molded body prepared by filling granules into a specific mold and pressing the granules into a specific shape, and d2 is the density of alumina (with no internal voids).

A dental alumina sintered body of the present invention encompasses not only sintered bodies of molded alumina particles sintered in the sintering step under atmospheric pressure and no applied pressure, but also sintered bodies compacted by a high-temperature pressing process such as HIP.

A certain embodiment of the method for producing a dental alumina sintered body is a method that does not involve a high-temperature pressing process such as HIP.

A greater relative density in a dental alumina sintered body of the present invention leads to fewer internal voids and less scattering of light. Because this results in superior aesthetics with increased translucency and total transmittance along with improved mechanical strength in a dental alumina sintered body of the present invention, it is preferable for a dental alumina sintered body of the present invention to exhibit higher relative density. A dental alumina sintered body of the present invention has a relative density of, for example, preferably higher than 95%, more preferably 98% or more, even more preferably 99.5% or more. Most preferably, a dental alumina sintered body of the present invention is essentially void free.

A smaller average crystal grain size in a dental alumina sintered body of the present invention is preferable because it leads to increased total transmittance in the sintered body.

The average crystal grain size in the alumina sintered body is preferably 0.3 to 8.0 µm. The average crystal grain size is more preferably 5 µm or less, even more preferably 3 µm or less, yet more preferably 2 µm or less, particularly preferably 1.5 µm or less.

In a dental alumina sintered body of the present invention, an average crystal grain size of 0.3 to 8.0 µm is preferable because it also leads to increased mechanical strength, translucency, and/or total transmittance.

The average crystal grain size of the dental alumina sintered body can be measured using the method described in the EXAMPLES below. The number of particles measured for determining the average crystal grain size in the dental alumina sintered body may be 100 or more, 200 or more, or 600 or more (for example, 600 or 800).

A dental alumina sintered body of the present invention has a total transmittance of preferably 40% or more, more preferably 42% or more, even more preferably 45% or more at a thickness of 1.0 mm.

When the total transmittance of the alumina sintered body is less than 40% at a thickness of 1.0 mm, it may lead to the inability to provide the translucency required for the incisal region of a dental prosthesis.

A dental alumina sintered body of the present invention has a total transmittance of preferably 65% or less, more preferably 60% or less, even more preferably 58% or less at a thickness of 1.0 mm.

The method of measurement of the total transmittance of the alumina sintered body is as described in the EXAMPLES below.

When the total transmittance of the alumina sintered body exceeds 65% at a thickness of 1.0 mm, the alumina sintered body may appear dark in the oral cavity after intraoral placement as a dental prosthesis, posing a risk of a notable reduction in the aesthetics of the dental prosthesis.

The type and content of alumina and the sintering aid containing a group 14 metallic element component in a dental alumina sintered body of the present invention are essentially the same as those in the composition and/or pre-sintered body before fabrication into the sintered body.

In view of superior durability as a dental material against impacts resulting from mastication and grinding during jaw movement in the oral environment, a dental alumina sintered body of the present invention has a biaxial flexural strength of preferably 580 MPa or more, more preferably 600 MPa or more, even more preferably 650 MPa or more, particularly preferably 700 MPa or more.

The biaxial flexural strength can be measured in compliance with JIS T 6526:2012, specifically, using the method described in the EXAMPLES below.

The sintered body has a b* value of preferably 0 or more, more preferably 0.1 or more, even more preferably 0.2 or more, particularly preferably 0.3 or more at a thickness of 1.2 mm.

The b* value is preferably less than 6.0, more preferably less than 4.0, even more preferably less than 3.5, particularly preferably less than 3.0.

The method of measurement of b* value is as described in the EXAMPLES below.

A dental alumina sintered body of the present invention may be a molded body of a predetermined shape. For example, the sintered body may have a disc (discotic) shape, a cuboidal shape, or a shape of a dental product (for example, a crown shape).

The methods of production of the alumina compositions, granules, powders, molded bodies, pre-sintered bodies, cut or ground workpieces, and sintered bodies described in the present invention are not limited those described in the embodiments above, and a variety of known methods are applicable, except where specified otherwise.

The present invention encompasses embodiments combining all or part of the foregoing features in various ways within the technical idea of the present invention, provided that the present invention can exhibit its effects.

### EXAMPLES

The present invention is described below in greater detail through the Examples.

It should be noted, however, that the present invention is in no way limited by the following Examples, and various changes may be made by a person with ordinary skill in the art within the technical idea of the present invention.

### [Measurement of Average Primary Particle Diameter of Alumina Particles Contained in Pre-Sintered Body]

The pre-sintered bodies obtained in the Examples and Comparative Examples below were used to prepare surface images, using a scanning electron microscope (VE-9800 manufactured by Keyence Corporation under this trade name). For the measurement of particle diameter, the SEM image with indicated primary particles was binarized with image analysis software (Image-Pro Plus manufactured by Hakuto Co., Ltd. under this trade name), and particles were recognized from the field (region) after indicating grain boundaries on individual crystal grains in the image. In areas with unclear grain boundaries, a reduce filter was applied to the region until each region reduced to a single or multiple points, and Voronoi polygons were created by using these points as generators of Voronoi polygons. Lines were drawn that connect the midpoints between two adjacent generators, and these lines were superimposed on the original particle image to separate adjacent particles. For example, in the case where image processing produced a particle that looks like a gourd, the particle was separated into two by assuming that two adjoining circular particles are seen as a single particle. In a processing file recognizing primary particle diameter, "Diameter" was selected from "Count/size dialog" to find the distribution (n = 4). Specifically, a mean value of particle diameters (primary particle diameters) measured in each field with the image analysis software (Image-Pro Plus) was determined for four fields from one sample.

### [Measurement of Vickers Hardness of Pre-Sintered Body]

The dental alumina pre-sintered bodies obtained in the Examples and Comparative Examples below were used to prepare samples measuring 18 mm in diameter and 1.5 mm in thickness, and measurements were conducted on these samples in compliance with JIS Z 2244:2020. Using a FALCON 500 manufactured by Innovatest, a load of 5 kgf was applied and maintained for 30 seconds, and the HV value was calculated (arithmetic mean value for n = 10). The results are presented in Table 1.

### [Measurement of Sintering Aid Content]

The dental alumina pre-sintered bodies and sintered bodies obtained in the Examples and Comparative Examples below were used as samples.

For the measurement, a field-emission scanning electron microscope (FE-SEM Reglus 8220, manufactured by Hitachi High-Tech Corporation) and an energy dispersive X-ray analyzer (Aztec Energy X-Max 50, manufactured by Oxford Instruments) were used under the following conditions. The measurement results for the dental alumina pre-sintered bodies are presented in Table 1. The sintering aid content in the sintered bodies is essentially the same as in the pre-sintered bodies.
Measurement magnification: 5,000×
Analysis mode: surface analysis
Accelerating voltage: 5 kV
Working distance: 15 mm ± 1 mm
X-ray extraction angle: 30 degrees
Dead time: 7%
Measurement time: 100 seconds

### [Measurement Method of Average Crystal Grain Size of Sintered Body]

The sintered bodies obtained in the Examples and Comparative Examples were photographed to capture surface images, using a scanning electron microscope (VE-9800 manufactured by Keyence Corporation under this trade name). The average crystal grain size was calculated through image analysis after indicating grain boundaries on individual crystal grains in the image.

For the measurement of average crystal grain size, the captured SEM image was binarized with image analysis software (Image-Pro Plus manufactured by Hakuto Co., Ltd. under this trade name), and particles were recognized from the field (region) by adjusting the brightness range to provide clear grain boundaries.

The crystal grain size from Image-Pro Plus is the average of the measurements of the length of a line segment connecting the contour line and passing through the center of gravity determined from the contour line of the crystal grain, conducted at 2-degree intervals with the center of gravity as the central point.

The measurement of crystal grain size was conducted for all particles not lying on the edges of the SEM photographic image (3 fields) of the dental alumina sintered body.

The average crystal grain diameter was calculated from the crystal grain size of each particle and the number of crystal grains. The resulting arithmetic average diameter was then determined as the average crystal grain size of the sintered body.

Note that "particles not lying on the edges of the image" refers to particles excluding those with contour lines extending beyond the frame of the SEM photographic image (particles with their contour lines interrupted by the boundary lines at the top, bottom, left, and right). To select the crystal grain size of all particles not lying on the edges of the image, the option in Image-Pro Plus was used that excludes all particles lying on the boundary lines. The results are presented in Table 2.

### [Measurement Method of Total Transmittance of Sintered Body]

The dental alumina sintered bodies of Examples and Comparative Examples were measured for total transmittance. The measurement was individually conducted for each layer of the alumina sintered bodies fabricated using the following method.

First, a molded body was fabricated from the raw material powder of each Example and Comparative Example by pressing the powder in a mold having a diameter of 30 mm. Here, the raw material powder was introduced into the mold in adjusted amounts to ensure the production of dental alumina sintered bodies with a thickness of 1.0 mm.

The molded body was heated from room temperature at 10°C/min. Following a 2-hour retention at 500°C for the purpose of debinding organic components, the molded body was heated at 10°C/min, and maintained for 6 hours at the highest pre-sintering temperature indicated in Table 1. The molded body was then gradually cooled at - 0.4°C/min to yield dental alumina pre-sintered bodies.

Subsequently, the alumina pre-sintered bodies were sintered for 2 hours at the highest sintering temperatures indicated in Table 2, fabricating alumina sintered bodies. The alumina sintered bodies were polished to a mirror finish on both sides to prepare alumina sintered bodies with a thickness of 1.0 mm. These alumina sintered bodies were then measured with a turbidimeter (Haze Meter NDH 4000 manufactured by Nippon Denshoku Industries Co., Ltd.). The measurement was conducted according to ISO 13468-1 and JIS K 7361-1:1997, and the arithmetic mean value was determined (n = 3). The results are presented in Table 2.

### [Measurement Method of Total Transmittance of Pre-Sintered Body]

The dental alumina pre-sintered bodies produced in the foregoing section [Measurement Method of Total Transmittance of Sintered Body] were machined into samples of 1.2 mm thickness, and these samples were measured using a turbidimeter (Haze Meter NDH 4000 manufactured by Nippon Denshoku Industries Co., Ltd.). The measurement was conducted according to ISO 13468-1 and JIS K 7361-1:1997, and the arithmetic mean value was determined (n = 3). The results are presented in Table 1.

### [Measurement Method of Biaxial Flexural Strength of Sintered Body]

The alumina sintered body obtained in each Example and Comparative Example was prepared into a sample measuring 15 mm in diameter and 1.2 mm in thickness. The sample sintered body was then measured for biaxial flexural strength in compliance with JIS T 6526:2012, using an Autograph (a universal precision testing machine manufactured by Shimadzu Corporation under the trade name AG-I 100kN) at a crosshead speed of 1.0 mm/min (arithmetic mean value for n = 5). The results are presented in Table 2.

The preferred biaxial flexural strength for the alumina sintered bodies is 580 MPa or more, more preferably 600 MPa or more, even more preferably 650 MPa or more, particularly preferably 700 MPa or more.

### [Measurement of Chromaticity (b* value) of Sintered Body]

The b* value was determined by measuring chromaticity (color space) in the L*a*b* color system (JIS Z 8781-4:2013 Color Measurements - Part 4: CIE 1976 L*a*b* color space) using a dental colorimeter (Crystaleye CE100-DC/JP, measurement mode: 7-band LED light source, manufactured by Olympus Corporation) with analysis software (Crystaleye, manufactured by Olympus Corporation). The arithmetic mean value was then calculated from the measured b* values (n = 3). The results are presented in Table 2.

The measurement was conducted using alumina sintered bodies measuring 14 mm in diameter and 1.2 mm in thickness.

The preferred b* value is 0 or more and less than 6.0, more preferably 0.1 or more and less than 4.0, even more preferably 0.2 or more and less than 3.5, particularly preferably 0.3 or more and less than 3.0.

### <Example 1>

After weighing 100 g of α-alumina raw material NXA-100 (manufactured by Sumitomo Chemical Co., Ltd.) and 0.0635 g of tin(IV) oxide (equivalent to 500 ppm by mass in terms of a Sn element), these were introduced into 0.7 L of ethanol and ultrasonically dispersed.

The mixture was then placed in a rotary container along with alumina beads, and the alumina raw material, including aggregated particles, was pulverized with a ball mill until the desired average primary particle diameter was achieved through mixing and deagglomeration.

The average primary particle diameter was measured by volume with ultrasonic waves being applied after a slurry diluted with ethanol was subjected to 30 minutes of ultrasonication, using a laser diffraction/scattering particle size distribution analyzer (manufactured by Horiba Ltd. under the trade name Partica LA-950). The desired slurry with hardly any secondary aggregation was obtained after about 1 hour of ball milling.

Subsequently, an organic binder was added to the slurry. An aqueous acrylic binder, serving as the organic binder, was added in an amount of 2 mass% with respect to the α-alumina raw material. The mixture underwent stirring with rotary vanes for 24 hours. After stirring, the slurry underwent drying and granulation with a spray dryer, yielding granules. The granules had an average particle diameter of 40 µm. The powder formed by these granules was poured into a cuboidal mold of a predetermined size, and uniaxially pressed at 150 MPa to yield a molded body.

The molded body obtained was placed in an electric furnace, and heated from room temperature at 10°C/min. Following a 2-hour retention at 500°C for the purpose of debinding organic components, the molded body was heated, maintained for 6 hours at a highest pre-sintering temperature of 750°C under atmospheric pressure, and gradually cooled at -0.4°C/min to yield a dental alumina pre-sintered body.

Subsequently, the dental alumina pre-sintered body was cut by machining to prepare a workpiece of 1.5 mm thickness. The workpiece was heated from room temperature at 10°C/min, retained at a highest sintering temperature of 1,400°C for 2 hours under atmospheric pressure, and gradually cooled at -0.4°C/min to yield a dental alumina sintered body.

In addition to the dental alumina sintered bodies prepared for the measurement methods described above, a disc-shaped dental alumina sintered body with a thickness of 5 mm was fabricated from the dental alumina pre-sintered body by modifying only the thickness, following the same procedure used for the 1.5 mm-thick workpiece. Visual inspection confirmed that yellow coloration was notably reduced even with a thickness of 5 mm.

### <Examples 2 to 4>

For Examples 2 to 4, dental alumina pre-sintered bodies and dental alumina sintered bodies were prepared following the same method used in Example 1, except that tin(IV) oxide was added in the amounts indicated in Table 1, and that the pre-sintering temperatures indicated in Table 1 were employed in Examples other than Example 2.

Additionally, disc-shaped dental alumina sintered bodies with a thickness of 5 mm were fabricated following the same method employed in Example 1. Visual inspection confirmed that yellow coloration was notably reduced even with a thickness of 5 mm.

### <Example 5>

A dental alumina pre-sintered body and a dental alumina sintered body were prepared following the same method used in Example 1, except that, in addition to tin(IV) oxide, magnesium chloride hexahydrate was added in an amount equivalent to 500 ppm by mass in terms of a Mg element, and that the pre-sintering temperature indicated in Table 1 was employed.

### <Example 6>

A dental alumina pre-sintered body and a dental alumina sintered body were prepared following the same method used in Example 1, except that, in addition to tin(IV) oxide, 300 ppm by mass of yttria-stabilized zirconia containing 4.5 mol% yttria was added with respect to alumina, and that the pre-sintering temperature indicated in Table 1 was employed.

Here, the yttria content in the yttria-stabilized zirconia refers to the mole percentage (mol%) of yttria relative to the total number of moles of zirconia and yttria.

Additionally, a disc-shaped dental alumina sintered body with a thickness of 5 mm was fabricated following the same method employed in Example 1. Visual inspection confirmed that yellow coloration was notably reduced even with a thickness of 5 mm.

### <Example 7>

For Example 7, a dental alumina pre-sintered body and a dental alumina sintered body were prepared following the same method used in Example 1, except that a different alumina raw material was used as indicated in Table 1, and that tin(IV) oxide was added in the amount indicated in Table 1.

Additionally, a disc-shaped dental alumina sintered body with a thickness of 5 mm was fabricated following the same method employed in Example 1. Visual inspection confirmed that yellow coloration was notably reduced even with a thickness of 5 mm.

### <Example 8>

For Example 8, a dental alumina pre-sintered body and a dental alumina sintered body were prepared following the same method used in Example 1, except that K₂SnO₃·3H₂O was added in the amount indicated in Table 1 in terms of a Sn equivalent, instead of tin(IV) oxide.

Additionally, a disc-shaped dental alumina sintered body with a thickness of 5 mm was fabricated following the same method employed in Example 1. Visual inspection confirmed that yellow coloration was notably reduced even with a thickness of 5 mm.

### <Example 9>

For Example 9, a dental alumina pre-sintered body and a dental alumina sintered body were prepared following the same method used in Example 1, except that germanium dioxide was added in the amount indicated in Table 1 in terms of a Ge equivalent, instead of tin(IV) oxide.

Additionally, a disc-shaped dental alumina sintered body with a thickness of 5 mm was fabricated following the same method employed in Example 1. Visual inspection confirmed that yellow coloration was notably reduced even with a thickness of 5 mm.

### <Comparative Example 1>

A dental alumina pre-sintered body and a dental alumina sintered body were prepared following the same method used in Example 1, except that, instead of tin(IV) oxide, magnesium chloride hexahydrate was added in an amount equivalent to 500 ppm by mass in terms of a Mg element, and that the pre-sintering temperature and the highest sintering temperature indicated in Table 1 and Table 2, respectively, were employed.

In addition to the dental alumina sintered bodies prepared for the measurement methods described above, a disc-shaped dental alumina sintered body with a thickness of 5 mm was fabricated from the dental alumina pre-sintered body by modifying only the thickness, following the same procedure used for the 1.5 mm-thick workpiece. Visual inspection confirmed a pronounced overall yellow tint with a thickness of 5 mm.

### <Comparative Example 2>

A dental alumina pre-sintered body and a dental alumina sintered body were prepared following the same method used in Example 1, except that, instead of tin(IV) oxide, magnesium chloride hexahydrate was added in an amount equivalent to 1,000 ppm by mass in terms of a Mg element, and that the pre-sintering temperature indicated in Table 1 was employed.

### <Comparative Example 3>

A dental alumina pre-sintered body and a dental alumina sintered body were prepared following the same method used in Example 1, except that no sintering aid was added, and that the pre-sintering temperature and the highest sintering temperature indicated in Table 1 and Table 2, respectively, were employed.

In addition to the dental alumina sintered bodies prepared for the measurement methods described above, a disc-shaped dental alumina sintered body with a thickness of 5 mm was fabricated from the dental alumina pre-sintered body by modifying only the thickness, following the same procedure used for the 1.5 mm-thick workpiece. Visual inspection confirmed a pronounced overall yellow tint with a thickness of 5 mm.

### <Comparative Example 4>

A dental alumina pre-sintered body and a dental alumina sintered body were prepared following the same method used in Example 1, except that, instead of tin(IV) oxide, magnesium chloride hexahydrate was added in an amount equivalent to 1,000 ppm by mass in terms of a Mg element, and cobalt chloride hexahydrate (additive) was added in an amount equivalent to 50 ppm by mass in terms of a Co element, and that the pre-sintering temperature and the highest sintering temperature indicated in Table 1 and Table 2, respectively, were employed.

### <Comparative Example 5>

A dental alumina pre-sintered body and a dental alumina sintered body were prepared following the same method used in Example 1, except that the alumina raw material indicated in Table 1 was used, and that, instead of tin(IV) oxide, magnesium chloride hexahydrate was added in an amount equivalent to 500 ppm by mass in terms of a Mg element, and europium(III) oxide was added in an amount equivalent to 3,600 ppm by mass in terms of a Eu element. The pre-sintering temperature and the highest sintering temperature indicated in Table 1 and Table 2, respectively, were employed.

In addition to the dental alumina sintered bodies prepared for the measurement methods described above, a disc-shaped dental alumina sintered body with a thickness of 5 mm was fabricated from the dental alumina pre-sintered body by modifying only the thickness, following the same procedure used for the 1.5 mm-thick workpiece. Visual inspection confirmed a pronounced overall yellow tint with a thickness of 5 mm.

The results for Examples and Comparative Examples are presented in Tables 1 and 2.

**[Table 1]**

| | Main component (Alumina) | Sintering aid | | Additive | | Pre-sintering conditions | | Average primary particle diameter [nm] | Vickers hardness [HV] | Total transmittance [%] |
|---|---|---|---|---|---|---|---|---|---|---|
| | Type | Type | Amount ¹⁾ [ppm] | Type | Amount ¹⁾ [ppm] | Pressure conditions | Pre-sintering temp. [°C] | | | |
| Ex. 1 | NXA-100 | SnO2(IV) | 500 | - | - | Atmospheric pressure | 750 | 100 | 72 | 0.82% |
| Ex. 2 | NXA-100 | SnO2(IV) | 250 | - | - | Atmospheric pressure | 750 | 100 | 87 | 0.81% |
| Ex. 3 | NXA-100 | SnO2(IV) | 1000 | - | - | Atmospheric pressure | 600 | 100 | 64 | 0.57% |
| Ex. 4 | NXA-100 | SnO2(IV) | 2000 | - | - | Atmospheric pressure | 800 | 100 | 93 | 0.75% |
| Ex. 5 | NXA-100 | SnO2(IV) | 500 | - | - | Atmospheric pressure | 800 | 100 | 95 | 0.96% |
| | | MgO | 500 | | | | | | | |
| Ex. 6 | NXA-100 | SnO2(IV) | 500 | - | - | Atmospheric pressure | 800 | 100 | 88 | 0.62% |
| | | ZrO2+Y2O3 | 300³⁾ | - | - | | | | | |
| Ex. 7 | TM-DA ⁴⁾ | SnO2(IV) | 635 | - | - | Atmospheric pressure | 750°C | 100 | 77 | 0.62% |
| Ex. 8 | NXA-100 | K2SnO3·3H2O | 1000 | - | - | Atmospheric pressure | 750°C | 100 | 74 | 0.85% |
| Ex. 9 | NXA-100 | GeO2 | 1000 | - | - | Atmospheric pressure | 750°C | 100 | 69 | 0.54% |

**[Table 1] Continued**

| | Main component (Alumina) | Sintering aid | | Additive | | Pre-sintering conditions | | Average primary particle diameter [nm] | Vickers hardness [HV] | Total transmittance [%] |
|---|---|---|---|---|---|---|---|---|---|---|
| | Type | Type | Amount ¹⁾ [ppm] | Type | Amount ¹⁾ [ppm] | Pressure conditions | Pre-sintering temp. [°C] | | | |
| Com. Ex. 1 | NXA-100 | MgO | 500 | - | - | Atmospheric pressure | 800 | 100 | 65 | - |
| Com. Ex. 2 | NXA-100 | MgO | 1000 | - | - | Atmospheric pressure | 800 | 100 | 62 | - |
| Com. Ex. 3 | NXA-100 | - | None | - | - | Atmospheric pressure | 800 | 100 | 66 | - |
| Com. Ex. 4 | NXA-100 | MgO | 1000 | CoCl2 | 50 | Atmospheric pressure | 800 | 100 | 71 | - |
| Com. Ex. 5 | TM-DS-92M ²⁾ | MgO | 500 | - | - | Atmospheric pressure | 800 | 120 | - | - |
| | | Eu2O3 | 3600 | | | | | | | |

| | | | | | | | | | | |
|---|---|---|---|---|---|---|---|---|---|---|
| 1) The content is expressed in terms of a Sn equivalent, a Mg equivalent, a Co equivalent, a Ge equivalent, or a Eu equivalent (ppm by mass) except for ZrO2+Y2O3 of Example 6. 2) TM-DS-92M represents the high-purity alumina TAIMICRON, DS-92M grade manufactured by TAIMEI CHEMICALS Co., Ltd. under this trade name. 3) The amount of 4.5Y yttria-stabilized zirconia added with respect to alumina (ppm by mass). 4) TM-DA represents the low-temperature-sinterable high-purity alumina TAIMICRON, TM-DA grade manufactured by TAIMEI CHEMICALS Co., Ltd. under this trade name. | | | | | | | | | | |

**[Table 2]**

| | Firing conditions | | Average crystal grain size | Total transmittance | Biaxial flexural strength | b* value |
|---|---|---|---|---|---|---|
| | Pressure conditions | Highest sintering temp. | | | | [-] |
| | [-] | [°C] | [µm] | [%] | [MPa] | |
| Ex. 1 | Atmospheric pressure | 1450 | 1.2 | 52.3% | 720 | 2.97 |
| Ex. 2 | Atmospheric pressure | 1450 | 1.0 | 46.3% | 820 | 3.12 |
| Ex. 3 | Atmospheric pressure | 1450 | 1.0 | 50.3% | 700 | 2.88 |
| Ex. 4 | Atmospheric pressure | 1450 | 1.1 | 46.3% | 650 | 2.63 |
| Ex. 5 | Atmospheric pressure | 1450 | 1.1 | 61.1% | 620 | 5.76 |
| Ex. 6 | Atmospheric pressure | 1450 | 1.3 | 48.2% | 842 | 2.54 |
| Ex. 7 | Atmospheric pressure | 1450 | 1.7 | 47.3% | 615 | 2.38 |
| Ex. 8 | Atmospheric pressure | 1450 | 1.1 | 53.4% | 720 | 3.79 |
| Ex. 9 | Atmospheric pressure | 1450 | 1.3 | 48.6% | 628 | 4.22 |
| Com. Ex. 1 | Atmospheric pressure | 1400 | 0.8 | 57.3% | 520 | 6.01 |
| Com. Ex. 2 | Atmospheric pressure | 1450 | 1.0 | 66.1% | 440 | 5.40 |
| Com. Ex. 3 | Atmospheric pressure | 1325 | 0.8 | 32.2% | 840 | 6.05 |
| Com. Ex. 4 | Atmospheric pressure | 1400 | 1.8 | 43.6% | 430 | 0.80 |
| Com. Ex. 5 | Atmospheric pressure | 1425 | 1.3 | 52.2% | 573 | 6.15 |

As demonstrated above, it was confirmed in Examples 1 to 9 that the dental alumina sintered bodies obtained through sintering under atmospheric pressure exhibit reduced yellow coloration and excellent aesthetics suited for dental use while possessing superior mechanical strength along with translucency suited for dental applications.

The dental alumina sintered bodies produced from the dental alumina pre-sintered bodies of Examples 1 to 4 and Examples 6 to 9 exhibited excellent aesthetics with notably reduced yellow coloration even with a thickness of approximately 5 mm and without a colorant, despite that the dental alumina sintered bodies were produced without hot isostatic pressing or other such processes after sintering under atmospheric pressure.

### INDUSTRIAL APPLICABILITY

A dental alumina pre-sintered body of the present invention can be used to provide a dental alumina sintered body having high total transmittance and a reduced yellow tint. A dental alumina pre-sintered body of the present invention becomes a highly aesthetic alumina sintered body suited for the appearance of the incisal edge, and is particularly suitable as a dental prosthesis for incisors or canines.

## Claims

1. A dental alumina pre-sintered body comprising alumina particles with an average primary particle diameter of 30 to 300 nm, and a sintering aid,
wherein the sintering aid comprises a group 14 metallic element component, and
the content of the group 14 metallic element component is more than 0 ppm and 5,000 ppm or less.

2. The dental alumina pre-sintered body according to claim 1, wherein the group 14 metallic element component is a tin component.

3. The dental alumina pre-sintered body according to claim 2, wherein the tin component is an inorganic tin compound.

4. The dental alumina pre-sintered body according to claim 2 or 3, wherein the tin component is at least one selected from tin(II) oxide, tin(IV) oxide, tin(VI) oxide, tin(II) chloride, tin(IV) chloride, tin(II) iodide, tin(IV) iodide, diammonium hexachlorostannate, tin(IV) sulfide, tin(II) sulfide, tin(II) sulfate, tin(IV) diphosphate, tin(II) acetate, tin(II) tartrate, potassium stannate(IV) trihydrate, and tin(II) oxalate.

5. The dental alumina pre-sintered body according to claim 1 or 2, wherein the group 14 metallic element component is a metal oxide.

6. The dental alumina pre-sintered body according to claim 1 or 2, wherein the content of the group 14 metallic element component is more than 60 ppm.

7. The dental alumina pre-sintered body according to claim 1 or 2, wherein the alumina particles comprise α-alumina with a purity of 99.5% or more.

8. The dental alumina pre-sintered body according to claim 1 or 2, which has a total transmittance of 0.3 to 5% at a thickness of 1.2 mm.

9. A dental mill blank having a portion comprising the dental alumina pre-sintered body of claim 1 or 2.

10. The dental mill blank according to claim 9, wherein said portion is a portion with the highest translucency.

11. A dental alumina sintered body having an average crystal grain size of 0.3 to 8.0 µm and comprising a group 14 metallic element component,
wherein the content of the group 14 metallic element component is more than 0 ppm and 5,000 ppm or less.

12. The dental alumina sintered body according to claim 11, wherein the group 14 metallic element component is a tin component.

13. The dental alumina sintered body according to claim 12, wherein the tin component is at least one selected from tin(II) oxide, tin(IV) oxide, tin(VI) oxide, tin(II) chloride, tin(IV) chloride, tin(II) iodide, tin(IV) iodide, diammonium hexachlorostannate, tin(IV) sulfide, tin(II) sulfide, tin(II) sulfate, tin(IV) diphosphate, tin(II) acetate, tin(II) tartrate, potassium stannate(IV) trihydrate, and tin(II) oxalate.

14. The dental alumina sintered body according to claim 12, wherein the content of the group 14 metallic element component is more than 60 ppm.

15. The dental alumina sintered body according to claim 12 or 13, wherein the group 14 metallic element component is a metal oxide.

16. The dental alumina sintered body according to claim 12 or 13, which has a total transmittance of 40 to 65% at a thickness of 1.0 mm.

17. The dental alumina sintered body according to claim 12 or 13, which has a biaxial flexural strength of 580 MPa or more.

18. A method for producing a dental alumina pre-sintered body, comprising the step of allowing a group 14 metallic element component to uniformly adhere to alumina particles,
wherein the dental alumina pre-sintered body comprises alumina particles with an average primary particle diameter of 30 to 300 nm, and a group 14 metallic element component, and
the content of the group 14 metallic element component is more than 0 ppm and 5,000 ppm or less.

19. The method for producing a dental alumina pre-sintered body according to claim 18, which further comprises molding the alumina particles to obtain a molded body, and pre-sintering the molded body to obtain a pre-sintered body.

20. The method for producing a dental alumina pre-sintered body according to claim 19, wherein the molded body is pre-sintered at a temperature of 600 to 900°C.

21. A method for producing a dental alumina sintered body, comprising sintering the dental alumina pre-sintered body of claim 1 or 2 at 1,500°C or less under atmospheric pressure.
